(19) Europäisches Patentamt
European Patent Office
Office européen des brevets

(11) Numéro de publication: **0 586 501 B1**

(12) # FASCICULE DE BREVET EUROPEEN

(45) Date de publication de fascicule du brevet: **21.12.94**

(51) Int. Cl.5: **A61K 7/06**, A61K 7/48, A61K 47/42

(21) Numéro de dépôt: **92911733.1**

(22) Date de dépôt: **03.06.92**

(86) Numéro de dépôt internationale : **PCT/FR92/00489**

(87) Numéro de publication internationale : **WO 92/21318 (10.12.92 92/31)**

(54) **DERIVES N-ACYLES DE MELANGES D'ACIDES AMINES ISSUS D'HYDROLYSATS DE PROTEINES DE CEREALES ET LEURS APPLICATIONS.**

(30) Priorité: **03.06.91 FR 9106672**

(43) Date de publication de la demande: **16.03.94 Bulletin 94/11**

(45) Mention de la délivrance du brevet: **21.12.94 Bulletin 94/51**

(84) Etats contractants désignés: **AT BE CH DE DK ES FR GB GR IT LI NL SE**

(56) Documents cités:
**EP-A- 0 308 278**
**DE-A- 3 422 496**
**FR-A- 2 603 800**
**GB-A- 1 153 408**
**GB-A- 2 095 994**

**Patent abstracts of Japan, vol. 10, no. 328 (C383)(2384) 7 November 1986 & JP A 61137808 (Ajinomoto Co Ltd)**

(73) Titulaire: **GIVAUDAN-LAVIROTTE**
**50-56, rue Paul-Cazeneuve**
**F-69008 Lyon (FR)**

(72) Inventeur: **BERGER, Christian**
**8, chemin de Charrière-Blanche**
**F-69130 Ecully (FR)**
Inventeur: **GACON, Paul**
**21, rue Barthélémy-Thimonier**
**F-69100 Tassin-la-Demi-Lune (FR)**

(74) Mandataire: **Caen, Thierry Alain et al**
**L'AIR LIOUIDE,**
**Service Brevets & Marques,**
**75, Ouai d'Orsay**
**F-75321 Paris Cédex 07 (FR)**

EP 0 586 501 B1

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

## Description

La présente invention se rapporte à de nouveaux dérivés N-acylés de mélanges d'acides aminés issus de l'hydrolyse totale de protéines d'origine végétale et leurs sels, ainsi qu'à leur application dans le domaine de la cosmétique et de la détergence.

Actuellement les dérivés N-acylés de mélange d'acides aminés obtenus à partir de l'hydrolyse de protéines d'origine animale sont couramment employés à des fins cosmétiques.

Du fait de leur structure amphiphile ces lipoaminoacides polyacylés peuvent progresser rapidement à travers les premières couches cellulaires pour venir soit restaurer les structures déficientes soit libérer leurs constituants actifs au sein de l'épiderme.

Ces structures lipoaminoacides sont donc des vecteurs biologiques particulièrement intéressants à titre de régulateurs de la physiologie cutanée et s'avèrent appropriées à de multiples applications, en particulier en cosmétique.

Les sources de ces mélanges d'acides aminés sont pour l'essentiel aujourd'hui, le collagène, l'élastine, la kératine et la caséine.

Compte-tenu de leur origine animale, ces hydrolysats présentent malheureusement des risques potentiels de contamination par des agents pathogènes dont il serait souhaitable de s'affranchir ; les utilisateurs ayant de plus en plus fréquemment tendance à de détourner de composés issus de l'animal pour toute application destinée à l'homme.

Par ailleurs, la demande de brevet britannique n° 1.153.408 décrit des dérivés N-acylés de mélange d'acides aminés obtenus à partir de l'hydrolyse de protéine d'origine végétale. Cependant la nature précise de ces protéines n'est pas décrite. Or la demanderesse a pu constater que selon la nature du végétal dont est issue la protéine, les composés en résultant peuvent présenter des propriétés très différentes. Il a ainsi pu être remarquer que des dérivés N-acylés de mélanges d'acides aminés issus d'hydrolysats de protéines de légumineuse, tel le soja présentaient certains inconvénients.

Ainsi, des solutions de tels dérivés N-acylés, et notamment des solutions de sels de ces dérivés, présentent une coloration relativement importante et gênante dans certaines applications, notamment dans le domaine de la cosmétique. D'autre part, il est souvent nécessaire de situer le pH de compositions cosmétiques aux environs de 5,5, soit à un pH proche de celui de la peau, de sorte à diminuer leur agressivité vis-à-vis de la peau. Or il a pu être constater que des solutions contenant de tels dérivés N-acylés, issus de protéines de légumineuse, tel le soja, se troublaient dès que leur pH était inférieur à environ 6,8. Or une caractéristique importante et souvent recherchée que doivent présenter les compositions cosmétiques, est leur limpidité.

Par ailleurs, lesdits dérivés issus notamment de protéines de soja présentent un pouvoir viscosant relativement élevé en solution aqueuse, ce qui rend parfois difficile la préparation de certaines compositions, notamment des compositions cosmétiques, à l'aide de telles solutions.

La présente invention a alors pour objet de nouveaux dérivés N-acylés de mélanges d'acides aminés, issus de protéines végétales qui permettent l'obtention de solutions très peu colorées et stables même à un pH de l'ordre de 5,5 et présentant un pouvoir viscosant en solution aqueuse relativement faible.

Un autre objet de l'invention consiste en des compositions cosmétiques contenant lesdits dérivés, de telles compositions possédant une activité cosmétique comparable, voire supérieure à des compositions contenant des dérivés N-acylés de mélanges d'acides aminés issus d'hydrolysats de protéines animales.

Un troisième objet de la présente invention consiste en des compositions détergentes contenant lesdits dérivés N-acylés.

L'invention consiste donc en de nouveaux dérivés N-acylés de mélanges d'acides aminés de formule générale I :

$$R - CO - NH - \underset{\displaystyle R'}{\underset{|}{CH}} - COOH \qquad I$$

dans laquelle :

R représente un radical en $C_4$ - $C_{30}$, saturé ou insaturé, linéaire ou ramifié et

R' représente une chaîne principale d'acide aminé,

ainsi que les sels de ces dérivés de mélanges d'acides aminés, lesdits mélanges d'acides aminés étant issus d'hydrolysats de protéines de céréales.

Selon un aspect avantageux de la présente invention, R représente un radical en $C_7$ - $C_{19}$ et plus particulièrement R représente un radical en $C_7$, $C_{11}$ ou $C_{15}$ de sorte que le reste R - CO dans la formule I représente un radical capriloyle, lauroyle ou palmitoyle.

Les protéines d'où sont issus les mélanges d'acides aminés peuvent provenir de diverses espèces de céréales, tels le maïs, l'orge, ou, de préférence, le blé.

A titre d'exemple, la composition moyenne en acides aminés des protéines du blé est la suivante :

| | | | |
|---|---|---|---|
| - Alanine | 3.9 | Lysine | 2.0 |
| - Arginine | 5.0 | Méthionine | 2.0 |
| - Acide aspartique | 5.3 | Phénylalanine | 5.3 |
| - Cystine | 2.7 | Proline | 4.9 |
| - Acide glutamique | 35.2 | Sérine | 5.4 |
| - Glycine | 3.5 | Thréonine | 3.4 |
| - Histidine | 2.0 | Thryptophane | 1.0 |
| - Isoleucine | 4.1 | Tyrosine | 3.6 |
| - Leucine | 8.4 | Valine | 5.1 |

Lorsqu'ils se présentent sous forme de sels, les dérivés N-acylés de l'invention peuvent notamment être des sels métalliques ou des sels de bases organiques. Ainsi, la fonction acide de ces produits peut être salifiée par des cations de métaux alcalins tels le sodium ou le potassium ou alcalino-terreux tels le calcium ou le magnésium ou d'autres métaux, tels qe le cobalt, le fer, l'aluminium, le manganèse, le cuivre, le zinc ou par des bases organiques telles que l'arginine, la lysine, les mono, di et triéthanolamine, l'ornithine, l'histidine, la morpholine et la choline. Ladite fonction acide peut également être salifiée par l'ammoniaque.

Lorsqu'ils sont salifiés par le cation de l'aluminium, les dérivés N-acylés de l'invention peuvent se présenter sous la forme de sels d'aluminium monobasique ou dibasique.

Ces dérivés N-acylés d'acides aminés obtenus par hydrolyse de protéines de céréales peuvent être obtenus, de façon connue en soi, en deux étapes.

La première étape consiste en l'hydrolyse totale de la protéine de céréale, par chauffage à des températures comprises entre 60 et 130 ° C de ladite protéine de céréale placée dans un milieu aqueux acide.

L'acidification dudit milieu peut être obtenue par des acides forts tels que l'acide sulfurique ou l'acide chlorhydrique.

La seconde étape consiste en l'acylation de l'hydrolysat précédemment obtenu à l'aide de dérivés activés carboxyliques de formules RCOOH, R étant défini comme précédemment.

De tels dérivés sont par exemple l'anhydride symétrique de ces acides, ou des chlorures d'acide. L'opération s'effectue généralement à une température comprise entre 0 ° C et 100 ° C.

Les dérivés N-acylés obtenus peuvent être éventuellement purifiés par des méthodes telles que la cristallisation ou la chromatographie.

L'invention s'étend également aux sels des dérivés N-acylés des acides ami nés obtenus par hydrolyse totale de protéines de céréales. Ces sels peuvent être préparés par réaction desdits dérivés N-acylés avec des bases organique ou inorganique, ou avec des dérivés métalliques.

A titre de bases organiques ou inorganique pouvant entrer dans le cadre de la présente invention, on peut citer la potasse, la soude, la chaux, la magnésie, l'ammoniaque, la triéthanolamine, la diéthanolamine, la monoéthanolamine, la morpholine, la lysine, l'histidine, l'arginine, l'ornithine et la choline.

A titre de sels obtenus par réaction aves des dérivés métalliques, on peut citer les sels de zinc, d'aluminium, de cuivre, de cobalt, de fer et de manganèse.

La présente invention se rapporte également aux compositions cosmétiques contenant au moins un dérivé N-acylé des acides aminés obtenus par hydrolyse totale de protéines de céréales ou un sel desdits dérivés, tels que décrits précédemment.

A titre de compositions cosmétiques, on peut citer les crèmes, les laits, les mousses, les aérosols, les gels, les sticks, les huiles, les émulsions, les shampooings, les savons, les pâtes dentifrices ainsi que les lotions aqueuses ou hydroalcooliques.

Dans lesdites compositions cosmétiques, les dérivés N-acylés précités et/ou les sels de ces dérivés sont présents dans des proportions de 0,1 à 50% et de préférence de 0,5 à 20% en poids par rapport au poids total de la composition. Dans ces compositions cosmétiques, les dérivés N-acylés de l'invention peuvent notamment être utiles en tant qu'émollient ou régulateur du pH de la peau.

L'invention concerne également des compositions détergentes contenant au moins un dérivé N-acylé des acides aminés et/ou un sel, de ces dérivés, tels que définis ci-dessus. Lesdits dérivés et/ou leurs sels sont présents dans des proportions de 0,1 à 50% en poids, plus généralement de 10 à 30% en poids par rapport au poids total de la composition.

Dans le cadre de la présente invention, on entend par composition détergente des compositions à usage autre que cosmétique. De telles compositions détergentes peuvent être notamment des compositions pour le lavage des textiles et de la vaisselle, ou des compositions pour le nettoyage et l'entretien des surfaces, en particulier des sols.

Ces compositions détergentes selon l'invention peuvent plus spécialement convenir au lavage des textiles délicats, par exemple en laine, en soie, en coton, ou en lin.

Les exemples suivants ont pour but d'illustrer la présente invention :

Exemple 1 :

Préparation du sel de sodium du dérivé N-lauroylé des acides aminés obtenus par hydrolyse totale de la protéine de blé

Un mélange constitué de 357g de protéine de blé, 225 $cm^3$ d'eau et 450 $cm^3$ d'acide chlorhydrique à 30% poids/poids est porté pendant 8 heures à reflux. Après refroidissement, 225 $cm^3$ d'eau sont ajoutés à la masse réactionnelle qui est ensuite ajustée à pH 3,5 avec 380 $cm^3$ de soude à 30% poids/poids. On ajoute alors 20g de noir décolorant, on laisse une heure en contact sous agitation, puis on filtre la suspension.

La solution aqueuse est ensuite portée à pH 10,5 à l'aide de 310 $cm^3$ de soude à 30% poids/poids, puis chauffée à 40 - 45 °C sous vide ($8.10^2$ Pa) pendant 4 heures; la masse réactionnelle est ensuite ajustée à pH 3,5 avec 150 $cm^3$ d'acide chlorhydrique à 30% poids/poids, et traitée à l'aide de 20g de noir décolorant pendant 20 minutes sous agitation. Après filtration on obtient 2285 g d'hydrolysat de protéine de blé.

A 500 g de cet hydrolysat, on ajoute 150 $cm^3$ d'eau, on porte la température à 30 °C puis on ajuste le pH à 10.5 avec 60 $cm^3$ de soude 30 % poids/poids, puis on coule simultanément, sous agitation, 71,5 g de chlorure de lauroyle et 40 $cm^3$ de soude 30 % poids/poids en maintenant le pH à 10.5 ± 0.3 et la température au dessous de 40 °C, en 1 heure environ. Après la fin d'addition, la température est maintenue à 40 °C pendant 30 minutes, puis à 60 °C pendant 1 heure. On élève alors la température à 80 °C puis on ajoute 115 $cm^3$ d'acide chlorhydrique 30% poids/poids, de manière à porter le pH vers 1. L'agitation est alors arrêtée et la phase aqueuse inférieure est soutirée. A la phase organique, on ajoute, sous agitation, 300g d'eau et on porte le mélange à pH 7 avec 25 $cm^3$ de soude 30% poids/poids. La solution est traitée à 70 °C avec 10g de noir décolorant, puis filtrée et l'on obtient ainsi 450g d'une solution aqueuse à 30% de sel de sodium du dérivés N-lauroylé des acides aminés obtenus par hydrolyse totale de la protéine de blé, sous forme d'une solution limpide, visqueuse, jaune à jaune ambrée.

Exemple 2

A titre comparatif, on prépare une solution à 22% d'un sel de sodium du dérivés N-lauroylé des acides aminés obtenus par hydrolyse totale de la protéine de soja, en procédant comme dans l'exemple 1 mais en substituant une protéine de soja à la protéine de blé.

La mesure de la coloration de cette solution à 22%, obtenue à partir de protéines de soja, montre une coloration de 30 Hazen, alors que la solution de l'exemple 1, à 30% de matière active, obtenue à partir d'une protéine de blé montre une coloration de 20 Hazen seulement.

Par ailleurs, la viscosité de ces deux solutions, mesurée par capilarité, est de 260 mPa.s ; une solution à 30% en poids de matière active selon l'invention permet donc l'obtention d'une viscosité identique à celle obtenue avec une solution à 22% en poids d'une matière active issue d'une protéine de soja.

On montre ainsi que les composés de l'invention conduisent à une viscosité faible.

Enfin, la solution obtenue à partir d'une protéine de blé reste limpide jusqu'à des valeurs de pH de l'ordre de 5,6. Au contraire, la solution obtenue à partir d'une protéine de soja se trouble dès que le pH est diminué au-dessous d'une valeur de l'ordre de 6,8.

Exemples 3 à 7 :

Ces exemples sont relatifs à des compositions cosmétiques selon l'invention.

Exemple 3 : Crème anti-acnéique

Formule :

| Phase | Sous-phase | Constituant | Quantité |
|---|---|---|---|
| III | I | Glycérides saturés de $C_{10}$ à $C_{18}$ polyoxyéthylénés glycolysées......... | 5 |
| | | Monostéarate glycérol................ | 3 |
| | | Polyoxypropylène (2) myristyl éther propionate.......................... | 3 |
| | | Polydiméthylsiloxane................. | 2 |
| | | Cholécithine........................ | 2 |
| | II | Mono, di palmito-stéarate de poly éthylène glycols.................... | 12 |
| | | Huile d'amande douce................ | 2 |
| | | Dérivé capryloylé des acides aminés de protéines de blé.......... | 2 |
| | IV | Conservateur....................... | 0,08 |
| | | Glycérine.......................... | 2 |
| | | Eau déminéralisée.................. | 61,62 |
| | | NaOH 30 %.......................... | 0,8 |
| | V | Extrait d'écorces d'oranges amères (concentré pour sirop).............. | 0,5 |
| | | Complexe collagène-glycosamino-glycanes........................... | 2 |
| | | Eau déminéralisée.................. | 2 |

Mode opératoire

La cholécithine est dissoute dans les constituants de la phase I et l'ensemble refroidi à 75 ° C. On ajoute les autres constituants de la phase grasse (II). Les phases III et IV sont chauffées isolément à 75 ° C, IV est versée dans III, l'ensemble est neutralisé avec NaOH 30 % puis refroidi à 30 ° C. On ajoute ensuite la phase V.

Exemple 4 : Crème anti-irritante et calmante

Formule :

| | | |
|---|---|---|
| I | Mono-distéarate de glycol et stéarate de polyoxyéthylène......... | 7 |
| | Dérivé palmitoylé des acides aminés de protéines de blé.......... | 4 |
| | Huile de noyaux..................... | 5 |
| | Isostéarate d'isostéaryle........... | 3 |
| | Polydiméthylsiloxane................ | 3 |
| | Huile de vaseline................... | 2 |
| II | Eau déminéralisée................... | 71,9 |
| | Conservateur........................ | 0,3 |
| | Glycérine........................... | 2 |
| | Extrait fluide de Grindélia......... | 0,5 |
| | Acide hyaluronique.................. | 1 |
| | α Bisabolol......................... | 0,2 |
| | Parfum.............................. | 0,1 |

Les phases I et II sont chauffées isolément à 75 °C. La phase II est ensuite versée dans I et l'ensemble émulsionné à la turbine pendant 5 min. Le mélange est laissé à refroidir lentement et les trois principes actifs restant y sont incorporés à 40 °C.

Exemple 5 : Crème de protection normale

Formule :

| | | |
|---|---|---|
| I | Mono-distéarate de 1,2 propylène glycol | 7 |
| | Dérivé palmitoyle des acides aminés de protéines de blé | 2 |
| | Huile d'avocat | 1 |
| | Huile d'amande douce | 2 |
| | Cire d'abeille | 0,5 |
| | Myristate de 2 octyldodécyl | 2 |
| | Filtre solaire U.V.B | 1 |
| II | Eau déminéralisée | 83,32 |
| | Gomme Xanthane | 0,3 |
| | Conservateur | 0,08 |
| | NaOH 30% | 0,1 |
| | Acétate de vitamine E | 0,5 |
| | Parfum | 0,2 |

pH : 6,75

On prépare une dispersion aqueuse de la gomme xanthane. Cette dispersion est chauffée en présence d'un conservateur à 75°C de même que la phase I. La phase II est ensuite versée dans la phase I et le mélange ainsi obtenu est traité avec de la soude à 30%. L'ensemble est refroidi à 30°C avant d'y incorporer l'acétate de vitamine E et le parfum.

Exemple 6 : Shampooing Bébé

Formule :

| Phase | Composant | Quantité |
|---|---|---|
| A | Lauryléthersulfate de sodium......... | 12 |
| | Dérivé lauroylé des acides aminés de protéines de blé, sel de sodium, (solution à 30% dans l'eau).......... | 20 |
| | Glycérylstéarate de polyéthylène glycol 200.......................... | 10 |
| | Distéarate de polyéthylèneglycol 6000................................ | 0,5 |
| | Parahydroxybenzoate de propyle sodé................................ | 0,15 |
| | Eau déminéralisée......... | q.s pour 100 g |
| B | Monolaurate de Sorbitan éthoxylé.... | 3 |
| | Parfum............................ | 0,15 |
| C | Solution de colorant hydrosoluble à 1%............................ | 0,1 |

Mode opératoire :

La phase A est chauffée à 70 °C et maintenue à cette température sous agitation pendant 20 min. Elle est ensuite refroidie jusqu'à 30 °C, de manière à y ajouter B préalablement tiédi à 30 °C. C est ajouté au mélange A et B.

Exemple 7 : Shampooing cheveux normaux

Formule :

| | | |
|---|---|---|
| A | Lauryléthersulfate de sodium......... | 25 |
| | Di-sodium nonxynol-10-sulfosuccinate........... | 10 |
| | Dérivé lauroylé des acides aminés de protéine de blé, sel de sodium (solution à 30% dans l'eau).......... | 10 |
| | NaCl................................ | 3 |
| | Distéarate de polyéthylèneglycol 6000................................ | 1 |
| | Parahydroxybenzoate de méthyle sodé.. | 0,18 |
| | Parahydroxybenzoate de propyle sodé.. | 0,04 |
| | Eau................................ | 47,13 |
| B | Monolaurate de sorbitan éthoxylé.... | 1,5 |
| | Parfum........................... | 0,15 |
| C | Extrait glycolique de camomille.... | 2 |

Ce shampooing est réalisé selon un mode opératoire identique à celui décrit en exemple 6.

**Revendications**

**Revendications pour les Etats contractants suivants : AT, BE, CH, LI, DE, DK, FR, GB, GR, IT, NL, SE**

**1.** Dérivés N-acylés de mélanges acide aminés de formule générale I :

$$R - CO - NH - \underset{\displaystyle R'}{\underset{|}{CH}} - COOH$$

dans laquelle :
R représente un radical en $C_4$ - $C_{30}$, saturé ou insaturé, linéaire ou ramifié
R' représente une chaîne principale d'acide aminé,
ainsi que les sels de ces dérivés de mélange d'acide aminés, lesdits mélanges d'acides aminés étant issus d'hydrolysats de protéines de céréales.

**2.** Dérivés N-acylés selon la revendication 1 caractérisés en ce que R représente un radical en $C_7$ - $C_{19}$.

**3.** Dérivés N-acylés selon l'une des revendications 1 et 2 caractérisés en ce que R représente un radical en $C_7$, $C_{11}$ ou $C_{15}$, de sorte que le reste R-CO dans ladite formule I, représente un radical capriloyle, lauroyle ou palmitoyle.

**4.** Dérivés N-acylés selon l'une des revendications 1 à 3 caractérisés en ce que lesdits sels sont des sels métalliques ou des sels de bases organiques.

**5.** Dérivés N-acylés selon la revendication 4 caractérisés en ce que lesdits sels métalliques sont les sels de métaux alcalins tels le sodium ou le potassium ou les sels de métaux alcalino-terreux, tels le calcium ou le magnésium.

**6.** Dérivés N-acylés selon l'une des revendications 1 à 5, caractérisés en ce que ladite protéine est une protéine de blé.

**7.** Composition cosmétique caractérisée en ce qu'elle contient au moins un dérivé N-acylé d'un mélange d'acides aminés de formule I et/ou un sel d'un de ces dérivés, tels que définis dans l'une quelconque des revendications 1 à 6.

**8.** Composition cosmétique selon la revendication 7 caractérisée en ce qu'elle contient de 0,1 à 50% en poids de préférence de 0,1 à 20% en poids dudit dérivé N-acylé et.ou dudit sel, par rapport au poids total de la composition.

**9.** Composition cosmétique, selon l'une des revendications 7 et 8 caractérisée en ce qu'elle se présente sous forme de crèmes, de laits, de mousses, d'aérosols, de gels, de sticks, d'huiles, d'émulsions, de savons, de shampooings, de pâtes dentifrices ou de lotions aqueuses ou alcooliques.

**10.** Utilisation à titre de produits cosmétiques des dérivés N-acylés de mélanges d'acides aminés de formule I et de leur sels tels que définis dans l'une des revendications 1 à 6.

**11.** Composition détergente caractérisée en ce qu'elle contient au moins un dérivé N-acylé d'un mélange d'acides aminés de formule I et/ou d'un sel d'un de ces dérivés tels que définis dans l'une quelconque des revendications 1 à 6.

**12.** Composition détergente selon la revendication 11 caractérisée en ce qu'elle contient de 0,1 à 50% en poids, de préférence de 10 à 30 % en poids dudit dérivé N-acylé et/ou dudit sel par rapport au poids total de la composition.

**13.** Composition détergente selon l'une des revendications 11 et 12 caractérisée en ce qu'elle consiste en une composition pour le lavage des textiles ou de la vaisselle, ou une composition pour le nettoyage et l'entretien des surfaces.

**Revendications pour l'Etat contractant suivant : ES**

**1.** Procédé de préparation de dérivés N-acylés de mélanges d'acides aminés de formule générale I :

$$R - CO - NH - \underset{\displaystyle R'}{\underset{|}{CH}} - COOH \qquad (I)$$

dans laquelle :

R représente un radical en $C_4$-$C_{30}$, saturé ou insaturé, linéaire ou ramifié,
R' représente une chaîne principale d'acide aminé,
ainsi que les sels de ces dérivés de mélange d'acides aminés,
ledit procédé étant caractérisé en ce que :

- on procède à l'hydrolyse totale d'une protéine de céréale de sorte à obtenir un mélange d'acides aminés, puis
- on acyle les acides aminés de l'hydrolysat à l'aide de dérivés carboxyliques activés de formule RCOOH, R étant tel que défini ci-dessus et,
- le cas échéant, on fait réagir les acides aminés N-acylés obtenus avec une base minérale ou organique.

**2.** Procédé selon la revendication 1, caractérisé en ce que R représente un radical en $C_7$-$C_{19}$.

**3.** Procédé selon l'une des revendications 1 et 2, caractérisé en ce que R représente un radical $C_7$, $C_{11}$ ou $C_{15}$, de sorte que le reste R-CO dans ladite formule I, représente un radical capriloyle, lauroyle ou palmitoyle.

**4.** Procédé selon l'une des revendications 1 à 3, caractérisés en ce que lesdits sels sont des sels métalliques ou des sels de bases organiques.

**5.** Procédé selon la revendication 4, caractérisé en ce que lesdits sels métalliques sont les sels de métaux alcalins tels le sodium ou le potassium ou les sels de minéraux alcalino-terreux, tels le calcium ou le magnésium.

**6.** Procédé selon l'une des revendications 1 à 5, caractérisé en ce que ladite protéine est une protéine de blé.

**7.** Composition cosmétique, caractérisée en ce qu'elle contient au moins un dérivé N-acylé d'un mélange d'acides aminés de formule I et/ou un sel d'un de ces dérivés, tels qu'obtenus selon le procédé de l'une quelconque des revendications 1 à 6.

**8.** Composition cosmétique selon la revendication 7, caractérisée en ce qu'elle contient de 0,1 à 50 % en poids, de préférence de 0,1 à 20 % en poids dudit dérivé N-acylé et/ou dudit sel par rapport au poids total de la composition.

**9.** Composition cosmétique selon l'une des revendications 7 et 8, caractérisée en ce qu'elle se présente sous forme de crèmes, de laits, de mousses, d'aérosols, de gels, de sticks, d'huiles, d'émulsions, de savons, de shampooings, de pâtes dentifrices ou de lotions aqueuses ou alcooliques.

**10.** Utilisation à titre de produits cosmétiques des dérivés N-acylés de mélanges d'acides aminés de formule I et de leurs sels tels qu'obtenus selon le procédé de l'une des revendications 1 à 6.

**11.** Composition détergente, caractérisée en ce qu'elle contient au moins un dérivé N-acylé d'un mélange d'acides aminés de formule I et/ou d'un sel d'un de ces dérivés tels qu'obtenus selon le procédé de l'une quelconque des revendications 1 à 6.

**12.** Composition détergente selon la revendication 11, caractérisée en ce qu'elle contient de 0,1 à 50 % en poids, de préférence de 10 à 30 % en poids dudit dérivé N-acylé et/ou dudit sel par rapport au poids total de la composition.

**13.** Composition détergente selon l'une des revendications 11 et 12, caractérisée en ce qu'elle consiste en une composition pour le lavage des textiles ou de la vaisselle, ou une composition pour le nettoyage et l'entretien des surfaces.

**Claims**

**Claims for the following Contracting States : AT, BE, CH, LI, DE, DK, FR, GB, GR, IT, NL, SE**

**1.** N-acyl derivatives of mixtures of amino acids of the general formula I :

$$R - CO - NH - \underset{\displaystyle R'}{\underset{|}{CH}} - COOH \qquad (I)$$

in which :
R represents a saturated or unsaturated, linear or branched radical with $C_4$ - $C_{30}$,
R' represents a main chain of an amino acid,
as well as the salts of these derivatives of mixtures of amino acids, the said mixtures of amino acids

being derived from hydrolysates of cereal proteins.

2. N-acyl derivatives according to claim 1, characterized in that R represents a radical with $C_7$ - $C_{19}$.

3. N-acyl derivatives according to one of claims 1 and 2, characterized in that R represents a radical with $C_7$, $C_{11}$ or $C_{15}$, such that the residue R - CO in the said formula I represents a caprilyl, lauryl or palmityl radical.

4. N-acyl derivatives according to one of claims 1 to 3, characterized in that the said salts are metal salts or salts of organic bases.

5. N-acyl derivatives according to claim 4, characterized in that the said metal salts are salts of alkali metals such as sodium or potassium or salts of alkaline earth metals, such as calcium or magnesium.

6. N-acyl derivatives according to one of claims 1 to 5, characterized in that the said protein is a wheat protein.

7. Cosmetic composition characterized in that it contains at least one N-acyl derivative of a mixture of amino acids of formula I and/or a salt of one of these derivatives, such as defined in any one of claims 1 to 6.

8. Cosmetic composition according to claim 7, characterized in that it contains 0.1 to 50 % by weight and preferably 0.1 to 20 % by weight of the said N-acyl derivative and/or the said salt, with respect to the total weight of the composition.

9. Cosmetic composition, according to one of claims 7 and 8 characterized in that it is in the form of creams, milks, foams, aerosols, gels, sticks, oils, emulsions, soaps, shampoos, toothpastes or aqueous or alcoholic lotions.

10. Use as cosmetic products of N-acyl derivatives of mixtures of the amino acids of formula I and of their salts such as defined in one of claims 1 to 6.

11. Detergent composition characterized in that it contains at least one N-acyl derivative of a mixture of amino acids of formula I and/or a salt of one of these derivatives such as defined in any one of claims 1 to 6.

12. Detergent composition according to claim 11 characterized in that it contains 0.1 to 50 % by weight, preferably 10 to 30 % by weight of the said N-acyl derivative and/or of the said salt with respect to the total weight of the composition.

13. Detergent composition according to one of claims 11 and 12 characterized in that it consists of a composition for the washing of textiles or dishes, or a composition for the cleaning and maintenance of surfaces.

**Claims for the following Contracting State : ES**

1. Process for preparing N-acyl derivatives of mixtures of amino acids of the general formula I :

$$R - CO - NH - \underset{\displaystyle R'}{\underset{|}{CH}} - COOH \qquad (I)$$

in which :

R represents a saturated or unsaturated, linear or branched radical with $C_4$ - $C_{30}$,

R' represents a main chain of an amino acid, as well as the salts of these derivatives of mixtures of amino acids,

the said process being characterized in that :

- total hydrolysis is carried out of a cereal protein so as to obtain a mixture of amino acids and then
- the amino acids of the hydrolysate are acylated with the aid of active carboxylic acid derivatives of formula RCOOH, R being such as defined above and,
- if required, the N-acyl amino acids obtained are reacted with an inorganic or organic base.

**2.** Process according to Claim 1, characterized in that R represents a radical with $C_7$-$C_{19}$.

**3.** Process according to one of Claims 1 and 2, characterized in that R represents a radical with $C_7$, $C_{11}$ or $C_{15}$, such that the residue R - CO in the said formula I represents a caprilyl, lauryl or palmityl radical.

**4.** Process according to one of Claims 1 to 3, characterized in that the said salts are metal salts or salts of organic bases.

**5.** Process according to Claim 4, characterized in that the said metal salts are salts of alkali metals such as sodium or potassium or salts of alkaline earth metals, such as calcium or magnesium.

**6.** Process according to one of Claims 1 to 5, characterized in that the said protein is a wheat protein.

**7.** Cosmetic composition characterized in that it contains at least one N-acyl derivative of a mixture of amino acids of formula I and/or a salt of one of these derivatives, such as obtained according to the process of any one of Claims 1 to 6.

**8.** Cosmetic composition according to Claim 7, characterized in that it contains 0.1 to 50 % by weight, preferably 0.1 to 20 % by weight of the said N-acyl derivative and/or the said salt, with respect to the total weight of the composition.

**9.** Cosmetic composition, according to one of Claims 7 and 8 characterized in that it is in the form of creams, milks, foams, aerosols, gels, sticks, oils, emulsions, soaps, shampoos, toothpastes or aqueous or alcoholic lotions.

**10.** Use as cosmetic products of N-acyl derivatives of mixtures of the amino acids of formula I and of their salts such as obtained according to the process of one of Claims 1 to 6.

**11.** Detergent composition characterized in that it contains at least one N-acyl derivative of a mixture of amino acids of formula I and/or a salt of one of these derivatives such as obtained according to the process of any one of Claims 1 to 6.

**12.** Detergent composition according to Claim 11 characterized in that it contains 0.1 to 50 % by weight, preferably 10 to 30 % by weight of the said N-acyl derivative and/or of the said salt with respect to the total weight of the composition.

**13.** Detergent composition according to one of Claims 11 and 12 characterized in that it consists of a composition for the washing of textiles or dishes, or a composition for the cleaning and maintenance of surfaces.

**Patentansprüche**
**Patentansprüche für folgende Vertragsstaaten : AT, BE, CH, LI, DE, DK, FR, GB, GR, IT, NL, SE**

**1.** N-Acylderivate von Aminosäuremischungen der allgemeinen Formel I:

$$\begin{array}{c} R - CO - NH - \underset{\displaystyle R'}{\underset{|}{CH}} - COOH \end{array}$$

wobei:
R für ein gesättigtes oder ungesättigtes, lineares oder verzweigtes $C_4$ bis $C_{30'}$ - Radikal,
R' für eine Aminosäurenhauptkette steht,
sowie die Salze dieser Derivate von Aminosäuremischungen,
wobei die Aminosäuremischungen aus Getreideproteinhydrolysaten gewonnen sind.

**2.** N-Acylderivate nach Anspruch 1, dadurch gekennzeichnet, daß R für ein $C_7$ bis $C_{19}$ - Radikal steht.

**3.** N-Acylderivate nach Anspruch 1 oder 2, dadurch gekennzeichnet, daß R für ein $C_7$ bis $C_{11}$ - oder ein $C_{15}$ - Derivat steht, so daß der R-CO-Rest in der Formel I für ein Capriloyl-, Lauroyl- oder Palmitoyl-Radikal steht.

**4.** N-Acylderivate nach einem der Ansprüche 1 bis 3, dadurch gekennzeichnet, daß die Salze Metallsalze oder organische Basensalze sind.

**5.** N-Acylderivate nach Anspruch 4, dadurch gekennzeichnet, daß die Metallsalze alkalische Metallsalze, wie beispielsweise Natrium oder Kalium, oder Erdalkalimetallsalze, wie beispielsweise Kalzium oder Magnesium sind.

**6.** N-Acylderivate nach einem der Ansprüche 1 bis 5, dadurch gekennzeichnet, daß das Protein ein Getreideprotein ist.

**7.** Kosmetische Zusammensetzung, dadurch gekennzeichnet, daß sie zumindest ein N-Acylderivat von Aminosäuremischungen der Formel I und/oder ein Salz dieser Derivate nach einem der Ansprüche 1 bis 6 enthält.

**8.** Kosmetische Zusammensetzung nach Anspruch 7, dadurch gekennzeichnet, daß sie 0,1 bis 50 Gew.%, vorzugsweise 0,1 bis 20 Gew.% des N-Acylderivats und/oder des Salzes, jeweils bezogen auf das Gesamtgewicht der Zusammensetzung, enthält.

**9.** Kosmetische Zusammensetzung nach Anspruch 7 oder 8, dadurch gekennzeichnet, daß sie als Creme, als Milch, als Schaum, als Aerosol, als Gel, als Stift, als Öl, als Emulsion, als Seife, als Shampoon, als Zahnpasta oder als wässrige oder alkoholische Lösung vorliegt.

**10.** Verwendung der N-Acylderivate von Aminosäuremischungen der Formel I oder ihrer Salze nach einem der Ansprüche 1 bis 6 als Kosmetikprodukte.

**11.** Waschmittelzusammensetzung, dadurch gekennzeichnet, daß sie zumindest ein N-Acylderivat von Aminosäuremischungen der Formel I und/oder ein Salz dieser Derivate nach einem der Ansprüche 1 bis 6 enthält.

**12.** Waschmittel nach Anspruch 11, dadurch gekennzeichnet, daß es 0,1 bis 50 Gew.%, vorzugsweise 10 bis 30 Gew.% des N-Acylderivats und/oder des Salzes, jeweils bezogen auf das Gesamtgewicht der Zusammensetzung, enthält.

**13.** Waschmittel nach Anspruch 11 oder 12, dadurch gekennzeichnet, daß es aus einer Zusammensetzung zum Waschen von Textilien oder von Geschirr oder aus einer Zusammensetzung zum Trocknen und Pflegen von Oberflächen besteht.

**Patentansprüche für folgenden Vertragsstaat : ES**

**1.** Verfahren zur Herstellung von N-Acylderivaten von Aminosäuremischungen der allgemeinen Formel I:

$$R - CO - NH - \underset{\displaystyle R'}{\underset{|}{CH}} - COOH$$

wobei:
R für ein gesättigtes oder ungesättigtes, lineares oder verzweigtes $C_4$ bis $C_{30}$- Radikal steht, R' für eine Aminosäurenhauptkette sowie die Salze dieser Derivate von Aminosäuremischungen steht, dadurch gekennzeichnet, daß man ein Getreideprotein vollständig hydrolisiert, um ein Aminosäurengemisch zu erhalten, anschließend die Aminosäuren des Hydrolysats mittels aktiven Carboxylderivaten der Formel RCOOH, mit R wie oben definiert, acyliert, und ggfs. die erhaltenen N-acylierten Aminosäuren mit einer anorganischen oder organischen Base reagieren läßt.

**2.** Verfahren nach Anspruch 1, dadurch gekennzeichnet, daß R für ein $C_7$ bis $C_{19}$ - Radikal steht.

**3.** Verfahren nach Anspruch 1 oder 2, dadurch gekennzeichnet, daß R für ein $C_7$ bis $C_{11}$ - oder ein $C_{15}$ - Derivat steht, so daß der R-CO-Rest in der Formel I für ein Capriloyl-, Lauroyl- oder Palmitoyl-Radikal steht.

**4.** Verfahren nach einem der Ansprüche 1 bis 3, dadurch gekennzeichnet, daß die Salze Metallsalze oder organische Basensalze sind.

**5.** Verfahren nach Anspruch 4, dadurch gekennzeichnet, daß die Metallsalze alkalische Metallsalze, wie beispielsweise Natrium- oder Kaliumcarbonat, oder Erdalkalimetallsalze, wie beispielsweise von Kalzium oder Magnesium sind.

**6.** Verfahren nach einem der Ansprüche 1 bis 5, dadurch gekennzeichnet, daß das Protein ein Getreideprotein ist.

**7.** Kosmetische Zusammensetzung, dadurch gekennzeichnet, daß sie zumindest ein N-Acylderivat von Aminosäuremischungen der Formel I und/oder ein Salz dieser Derivate nach einem der Ansprüche 1 bis 6 enthält.

**8.** Kosmetische Zusammensetzung nach Anspruch 7, dadurch gekennzeichnet, daß sie 0,1 bis 50 Gew.%, vorzugsweise 0,1 bis 20 Gew.% des N-Acylderivats und/oder des Salzes, jeweils bezogen auf das Gesamtgewicht der Zusammensetzung, enthält.

**9.** Kosmetische Zusammensetzung nach Anspruch 7 oder 8, dadurch gekennzeichnet, daß sie als Creme, als Milch, als Schaum, als Aerosol, als Gel, als Stift, als Öl, als Emulsion, als Seife, als Shampoon, als Zahnpasta oder als wässrige oder alkoholische Lösung vorliegt.

**10.** Verwendung der N-Acylderivate von Aminosäuremischungen der Formel I oder ihrer Salze nach einem der Ansprüche 1 bis 6 als Kosmetikprodukte.

**11.** Waschmittelzusammensetzung, dadurch gekennzeichnet, daß sie zumindest ein N-Acylderivat von Aminosäuremischungen der Formel I und/oder ein Salz dieser Derivate nach einem der Ansprüche 1 bis 6 enthält.

**12.** Waschmittel nach Anspruch 11, dadurch gekennzeichnet, daß es 0,1 bis 50 Gew.%, vorzugsweise 10 bis 30 Gew.% des N-Acylderivats und/oder des Salzes, jeweils bezogen auf das Gesamtgewicht der Zusammensetzung, enthält.

**13.** Waschmittel nach Anspruch 11 oder 12, dadurch gekennzeichnet, daß es aus einer Zusammensetzung zum Waschen von Textilien oder von Geschirr oder aus einer Zusammensetzung zum Trocknen und Pflegen von Oberflächen besteht.